# EUROPÄISCHE PATENTSCHRIFT

(11) **EP 2 843 276 B1**
(45) Veröffentlichungstag und Bekanntmachung des Hinweises auf die Patenterteilung: **06.01.2016**
(21) Anmeldenummer: 13004234.4
(22) Anmeldetag: 28.08.2013
(51) Int. Cl.: F16K 11/085, A61M 16/10, A61M 16/20

(54) **Rotationsventil**
Rotary valve
Vanne rotative

(43) Veröffentlichungstag der Anmeldung: 04.03.2015
(73) Patentinhaber: Netzel, Thomas, 21509 Glinde (DE)
(72) Erfinder: Netzel, Thomas, 21509 Glinde (DE)
(74) Vertreter: Grättinger Möhring von Poschinger Patentanwälte Partnerschaft

(56) Entgegenhaltungen:
- WO-A1-86/01583
- US-A- 4 702 240
- US-A1- 2011 071 390

## Beschreibung

Die Erfindung betrifft ein Rotationsventil zur Flussrichtungsumkehr eines druckbeaufschlagten Fluidstroms sowie einen mechanischen In-/ Exsufflator zur Behandlung von Patienten mit Ateminsuffizienz, welches ein derartiges Rotationsventil umfasst.

Ein bekanntes Rotationsventil ist in der Deutschen Offenlegungsschrift DE 10247406 A1 beschrieben. Es regelt den Durchlass eines flüssigen oder gasförmigen Mediums mittels eines Ventilkörpers in Form eines drehbar gelagerten Zylinders, der im Zusammenwirken mit einem metallenen Dichtungsband den Durchlass des Mediums sperrt oder öffnet.

Neben der Verwendung eines derartigen Ventilkörpers bestehen keine Ähnlichkeiten zwischen dem bekannten Rotationsventil und den oben genannten Erfindungsgegenständen.

Ein Rotationsventil im Sinne der vorliegenden Anmeldung besteht prinzipiell aus dem eigentlichen Ventilkörper, welcher rotierend in einem Ventilgehäuse gelagert ist.

Bei der Regelung von druckbeaufschlagten Fluidströmen mit Flussrichtungsumkehr geht es wie im vorliegenden Fall darum, dass über das Rotationsventil eine Fördereinrichtung durchströmt wird, die mit konstanter Förderrichtung arbeitet, d.h. sie wird vom Fluidstrom stets in der gleichen Flussrichtung durchströmt. Auf diese Weise gelingt es, das generelle Strömungssystem insoweit von nachteiligen Strömungseinflüssen durch die Fördereinrichtung weitgehend zu verschonen.

Dies gilt keineswegs uneingeschränkt für druckbeaufschlagte Fluidströme mit hohem Volumendurchsatz, insbesondere für deren Flussrichtungsumkehr, wenn die Umschaltwege und die Umschaltzeiten kurz sind. Derartige Systeme neigen nicht nur zum Überschwingen und zu einer längeren Einstellzeit bis zum Erreichen eines definierten Drucks/Flusses während des Umschaltvorgangs. Darüber hinaus treten dynamische Strömungsvorgänge auf, welche durch die wechselnde Druckbeaufschlagung hinsichtlich ihrer Druckstabilität während der kurzen Phasen der Flussrichtungsumschaltung kaum beherrschbar sind.

Damit besteht für bestimmte Anwendungsfälle das Problem, dass gewollte Umschaltstöße infolge hoher Druckdynamik nicht hinreichend deutlich produzierbar sind.

Derartige Wirkungen sind beispielsweise erwünscht bei sogenannten mechanischen In-/ Exsufflatoren - gelegentlich auch als Sekretmobilisationsgerät bezeichnet - zur Behandlung von Patienten mit Ateminsuffizienz, wobei für den angestrebten Sekrettransport hustenartige Stoßbelastungen der Lunge erwünscht sind, während deren Einwirkung sich der Patient als Folge seiner Erkrankung weitgehend passiv verhält.

Demzufolge liegt der vorliegenden Erfindung die Aufgabe zugrunde, ein Rotationsventil der eingangs genannten Art zu schaffen, welches insbesondere bei Verwendung in einem mechanischen In-/ Exsufflator die oben erläuterten Nachteile vermeidet, aber geeignet ist, die gewollten therapeutischen Wirkungen zu erzeugen. Insbesondere geht es darum, die Wirkung des Umschaltstoßes bei der Flussrichtungsumkehr - auch bei schneller Abfolge - möglichst deutlich zu gewährleisten und dabei einen Sekrettransport für ein Fördervolumen in einem Bereich von 160 bis 300 l/min bei Umschaltfrequenzen von bis zu 5 Hz zu ermöglichen.

Ein weiteres Ziel der vorliegenden Erfindung besteht darin, ein Rotationsventil zu schaffen, welches einen hohen Volumenstrom im Rahmen definierter Leitungsverbindungen für flüssige und gasförmige Medien ermöglicht und dabei das Eindringen von Fremdfluid in das System verhindert.

Für die genannte therapeutische Anwendung eines mechanischen In-/ Exsufflators besteht eine besondere Anforderung an das dabei verwendete Rotationsventil darin, dass die nicht invasive Schleimmobilisierung durch schnell wechselnde Beaufschlagung der Atemwege mit Über- und Unterdruck entsprechend gefördert und die dabei auftretende Druckdynamik verbessert wird, so dass Sekretansammlungen im respiratorischen Trakt und das Wohlbefinden des Patienten während der Therapie durch störende Druckschwingungen weitgehend vermieden werden.

In technischer Hinsicht werden die erfindungsgemäßen Ziele und Wirkungen durch das Zusammenwirken eines neuartig gestalteten Ventilkörpers mit einem die Anschlüsse nach außen vermittelnden Ventilgehäuse erzielt, wobei der Ventilkörper zwischen zwei Schaltstellungen um seine Längsachse verschwenkbar im Ventilgehäuse gelagert ist.

Dabei ist eine Fördereinheit für den Fluidstrom je nach Schaltstellung des Ventilkörpers saugseitig in einer ersten Flussrichtung mit einem Frischfluidkanal und in einer zweiten Flussrichtung mit einem Wirkkanal, druckseitig in der ersten Flussrichtung mit dem Wirkkanal und in der zweiten Flussrichtung mit dem Frischfluidkanal, verbunden.

Das bedeutet für die genannte therapeutische Anwendung bei einem mechanischen In-/ Exsufflator, dass die erste Flussrichtung der Inhale-Schaltung entspricht, während die zweite Flussrichtung der Exhale-Schaltung entspricht.

Der Wirkkanal ist dabei für beide Flussrichtungen stets mit dem Patienten z.B. über eine Schlauchleitung und eine Atemmaske verbunden, in der ersten Flussrichtung als Luftstoß des Einatmens, in der zweiten Flussrichtung als Luftsog des Ausatmens.

Der Frischfluidkanal dient in der ersten Flussrichtung zum Ansaugen von Frischluft; in der zweiten Flussrichtung dient er zum Ausstoßen der verbrauchten Atemluft des Patienten.

Bezüglich der Ausbildung des Ventilkörpers im Einzelnen wird auf die Merkmale 1.c) und 1.d) in Patentanspruch 1 verwiesen.

Die Mittelbohrung des Ventilkörpers ist in beiden Flussrichtungen stets sogbeaufschlagt, wodurch es gelingt, unerwünschte Druckschwingungen während des Umschaltens der Flussrichtung weitgehend zu unterdrücken.

Einen wichtigen Beitrag zur Druckstabilität leistet ferner ein Hüllkanal auf der Außenseite des Ventilkörpers, der für beide Flussrichtungen die Umströmung des Ventilkörpers in entgegengesetzten Richtungen ermöglicht, nämlich im Inhale-Kreislauf von der Fördereinheit zur Wirköffnung des Ventilgehäuses hin bei relativ hohen Fluiddrücken und im Exhale-Kreislauf von der Fördereinheit zur Frischfluidöffnung des Ventilgehäuses hin bei relativ niedrigen Fluiddrücken.

Einen weiteren wichtigen Beitrag zur Strömungsberuhigung und zur Vermeidung unerwünschter Druckschwingungen leistet die jeweils zwischen beiden Schaltstellungen des Ventilkörpers überfahrene Bypassposition, wobei ein äußerer Bypass in der Hülle des Ventilkörpers eine Verbindung zwischen der Wirköffnung und der Frischfluidöffnung des Ventilgehäuses und ein innerer Bypass eine Verbindung zwischen dem Hüllkanal und der Saugseite der Fördereinheit bildet.

Dadurch, dass der äußere Bypass innerhalb eines Trennstegs des Ventilkörpers verläuft, durch welchen der Hüllkanal in beiden Umströmungsrichtungen begrenzt wird, werden nicht nur Druckschwankungen zwischen beiden Umströmungswegen unterbunden. Durch den äußeren Bypass gelingt überdies die Erzeugung eines Druckausgleichs zwischen Frischfluidöffnung einerseits und Wirköffnung andererseits, so dass der Patient auch während längerer Behandlungspausen gefiltertes Frischflüid frei atmen kann.

Durch den inneren Bypass wird eine Verbindung zwischen dem Hüllkanal und der Saugseite der Fördereinheit hergestellt, die es ermöglicht, dass während der Flussrichtungsumschaltung die Fördereinrichtung mit vermindertem Fluidvolumenstrom weiterläuft, wodurch sich eine Verbesserung der Druckstabilität beim Umschaltvorgang ergibt und wodurch sich zusätzliche Regeleingriffe zur Vermeidung störender Druckschwingungen erübrigen.

Bezüglich weiterer vorteilhafter Ausgestaltungen des Gehäusedeckels des Ventilgehäuses wird auf die Ansprüche 5 und 6 verwiesen. Demzufolge ist eine im Deckel angeordnete Saugleitung an die offene Seite der Mittelbohrung des Ventilkörpers angeschlossen. Ferner sind im Gehäusedeckel zum Ventilkörper hin vorspringende Anschlagflächen vorgesehen, die mit einem Gegenanschlag des Ventilkörpers zur Begrenzung der beiden Schaltstellungen zusammenwirken. Die Verschwenkung des Ventilkörpers in der jeweils gewünschten Frequenz erfolgt vorteilhaft mit Hilfe eines Stellmotors, welcher drehfest mit dem Ventilkörper an dessen dem Gehäusedeckel gegenüberliegendem Ende gekoppelt ist. Bezüglich weiterer vorteilhafter Ausgestaltungen der Erfindung wird auf die Unteransprüche 8 bis 10 verwiesen. Demnach ist ein Schwenkwinkel zwischen beiden Schaltstellungen des Ventilkörpers von etwa 90° vorteilhaft. Auch höhere Schaltfrequenzen bis zu 5 Hz sind damit möglich.

Bezüglich der Ausgestaltung eines mechanischen In-/ Exsufflators zur Behandlung von Patienten mit Ateminsuffizienz wird auf Patentanspruch 11 mit Unteransprüchen 12 bis 14 verwiesen. Ein derartiger mechanischer In-/ Exsufflator verwendet ein Rotationsventil mit Ventilkörper und Ventilgehäuse gemäß ein oder mehreren der Patentansprüche 1 bis 10, wobei das Ventilgehäuse an den Förderkreislauf einer mit gleichbleibender Drehrichtung rotierender Fluidfördereinheit angeschlossen ist und wobei dessen Ventilkörper zum Zwecke der Flussrichtungsumkehr mit einstellbarer Frequenz zwischen zwei Schaltstellungen verschwenkbar ist, derart, dass in einer Inhale-Flussrichtung die Saugseite der Fluidfördereinheit mit einer Frischfluidöffnung des Ventilgehäuses, deren Druckseite mit einer die Lunge des Patienten beaufschlagenden Wirköffnung des Ventilgehäuses verbunden ist und dass in einer Exhale-Flussrichtung die Saugseite der Fluidfördereinheit mit der Wirköffnung, deren Druckseite mit der Frischfluidöffnung verbunden ist.

Eine Besonderheit an dem erfindungsgemäßen Rotationsventil besteht darin, dass es einen zylindrischen Ventilkörper aufweist, welcher eine Mittelbohrung im Sinne eines axialen Fluidkanals in Form einer Kernsacklochbohrung besitzt, aus welcher zwei Fluidkanäle, ein Frischfluidkanal und ein Wirkkanal radial abzweigen; ferner weist der Ventilkörper einen äußeren Hüllkanal auf, der in Art eines Teilhüllkanals in den Umfangsbereich des zylindrischen Ventilkörpers eingebettet ist. Die radial abgehenden Fluidkanäle sind, abhängig von der Drehstellung des Ventilkörpers entweder mit der Wirköffnung oder der Frischfluidöffnung des Ventilgehäuses verbunden. Dadurch, dass die Mittelbohrung auf ihrer dem Gehäusedeckel zugewandten Seite gegenüber dem Ventilgehäuse abgedichtet ist, ergibt sich eine verbesserte Druckdynamik im Arbeitsbereich des Rotationsventils auch bei hohen Druckwerten auf der Wirkseite desselben.

Dabei ermöglicht die vorliegende Erfindung, dass die mit dem erfindungsgemäßen Rotationsventil konstant erzielbaren Drücke bei deutlich verkürzter Umschaltzeit liegen und somit einen demgegenüber verbesserten therapeutischen Effekt aufweisen.

Im Folgenden wird ein Ausführungsbeispiel der Erfindung anhand der Zeichnung erläutert. Es zeigen
Fig. 1 einen teilweise schematischen Querschnitt durch einen mechanischen In-/ Exsufflator
Fig. 2 und 3 unterschiedliche isometrische Darstellungen des Ventilkörpers
Fig. 4 einen Axialschnitt durch ein Rotationsventil in der Inhale-Position
Fig. 5 einen Querschnitt gemäß V-V, der Fig. 4
Fig. 6 einen Querschnitt gemäß VI-VI der Fig. 4
Fig. 7 einen Querschnitt gemäß VII-VII der Fig. 4
Fig. 8 einen Axialschnitt durch das Rotationsventil in der Exhale-Position
Fig. 9 einen Querschnitt gemäß IX-IX der Fig. 8
Fig. 10 einen Querschnitt gemäß X-X der Fig. 8

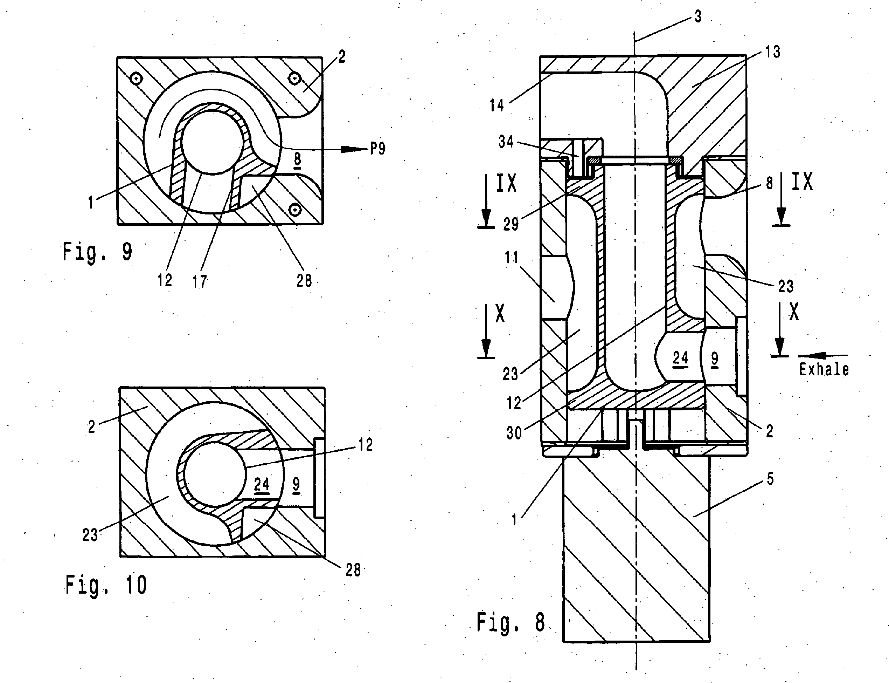

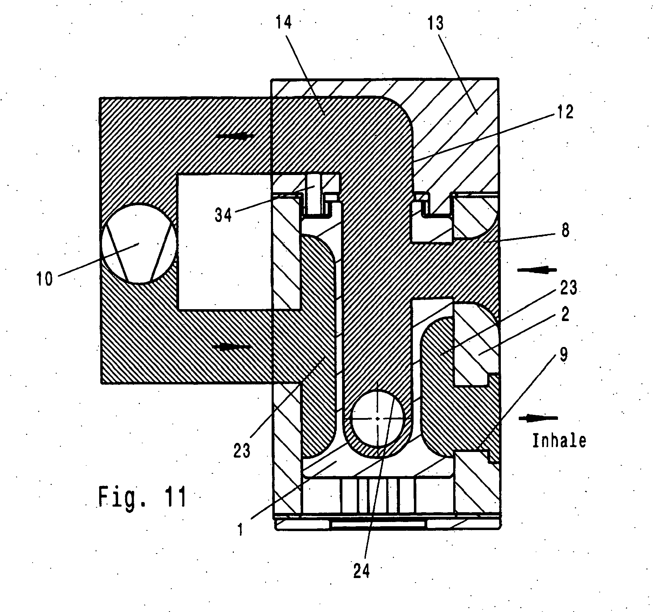

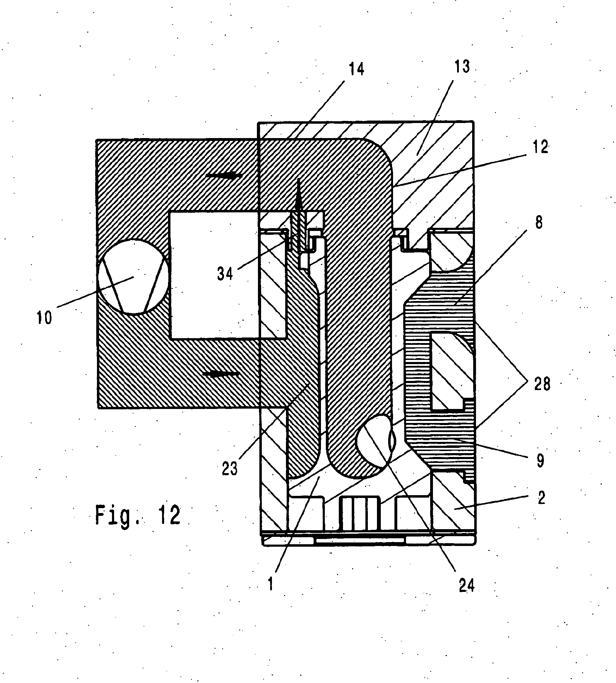

Fig. 10 bis 12 jeweils einen axialen Querschnitt durch einen mechanischen In-/ Exsufflator , welcher an eine Fördereinheit angeschlossen ist, in der Inhale-Position (Fig. 10), der Bypassposition (Fig. 11) und der Exhale-Position (Fig. 12).

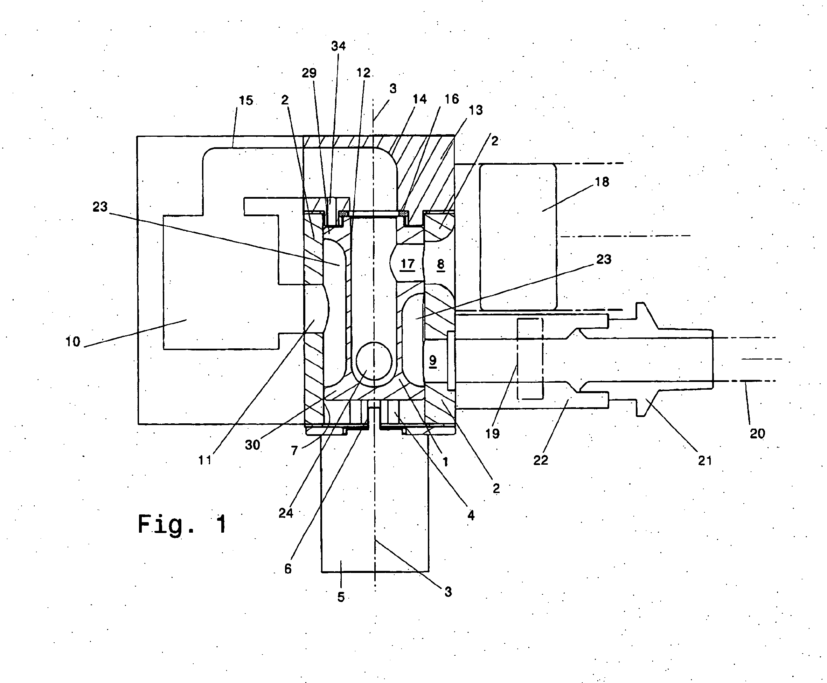

Fig. 1 zeigt einen teilweise schematischen Querschnitt durch einen mechanischen In-/ Exsufflator, wobei die Schnittebene durch die Längsachse eines Rotationsventils verläuft, im wesentlichen bestehend aus einem Ventilkörper 1 und einem Ventilgehäuse 2, in welchem der Ventilkörper um seine Längsachse 3 verschwenkbar aufgenommen ist. An seinem unteren Ende besitzt der Ventilkörper 1 Kupplungsvorsprünge 4, in deren Zwischenräume ein mit einem Stellmotor 5 fest verbundenes Kupplungsgegenstück 6 eingreift. Der Stellmotor 5 dient als Antrieb für eine oszillierende Schwenkbewegung des Ventilkörpers 1 um seine Längsachse 3.

Der Ventilkörper 1 ist in einer zylindrischen Bohrung 7 des Ventilgehäuses 2 um seine Längsachse 3 in beide Drehrichtungen verschwenkbar aufgenommen, so dass er mittels des Stellmotors 5 eine zwischen Anschlägen oszillierende Bewegung ausführen kann.

Das Ventilgehäuse 2 besitzt in seinem bezüglich der Zeichnung oberen Teil eine radiale Frischfluidöffnung 8 und in seinem unteren Teil eine radiale Wirköffnung 9, jeweils auf der rechten Bildseite. Auf der gegenüberliegenden Seite befindet sich eine mit einer Fluidfördereinheit 10 verbundene druckseitige Anschlussbohrung 11. Ein Gehäusedeckel 13 umfasst eine Saugleitung 14, welche die saugseitige Anschlussverbindung zur Fördereinheit 10 bildet. Ein strichpunktiert angedeuteter Saugkanal 15 verbindet die Saugleitung 14 mit dem saugseitigen Ende der Fördereinheit 10. Als geeignete Fördereinheit hat sich eine Turbine für fluide Medien der Firma Micronel, Typ U65H2 erwiesen.

Der Ventilkörper 1 besitzt eine Mittelbohrung 12, welche nach oben zur Saugleitung 14 hin offen ist, also ständig dem Sog der Fördereinheit 10 unterliegt. Mittels einer Ringdichtung 16 ist der Ventilkörper 1 mit seinem oberen Ende dichtend an den Gehäusedeckel 13 angeschlossen. Sowohl gegenüber der Ringdichtung 16 als auch gegenüber der zylindrischen Bohrung 7 des Ventilgehäuses ist die Außenkontur des Ventilkörpers mit geringer Minustoleranz bemessen, so dass die Schwenkbeweglichkeit des Ventilkörpers innerhalb der Gehäusebohrung 7 nicht gebremst wird und eine störende Leckage des transportierten Fluids unterbleibt. Sowohl der Ventilkörper 1 als auch das Ventilgehäuse 2 bestehen bevorzugt aus PE der Ausprägung UHMW (Ultra high molecular weight), gekennzeichnet durch hohen Abriebwiderstand, geringen Reibkoeffizient und hohe Formbeständigkeit auch bei hohen Temperaturen.

Bei der in Fig. 1 gezeigten Inhale-Position des Ventilkörpers 1 ist die Frischfluidöffnung 8 des Ventilgehäuses 2 an eine Frischfluidbohrung 17 des Ventilgehäuses angeschlossen, d.h. frisches Fluid wird von außen durch einen Luftfilter 18 angesaugt und über die Mittelbohrung 12 des Ventilkörpers 1, die Saugleitung 14 im Gehäusedeckel 13 und den Saugkanal 15 an das saugseitige Ende der Fördereinheit 10 weitergeleitet. Von dort wird es über die druckseitige Anschlussbohrung 11 des Ventilgehäuses 2 an den Patienten über die Wirköffnung 9 des Ventilgehäuses 2, einen daran angeschlossenen Oszillator 19 und eine Schlauchleitung 20 weitergeleitet, welche über einen Schlauchanschluss 21 an einen Gehäusestutzen 22 angeschlossen ist.

Der Luftfilter 18 dient als Partikelfilter für die angesaugte Frischluft, der Oszillator 19 zur Erzeugung von Schwingungen zum Zwecke der Sekretmobilisierung in der Lunge des Patienten. Der druckseitige Fluidvolumenstrom aus der Fördereinheit 10 umströmt den Ventilkörper 1 oberhalb der Zeichenebene, d.h. in der Zeichnung von oben gesehen entgegen den Uhrzeigersinn im Inneren eines in der Außenseite des Ventilkörpers 1 eingeformten Hüllkanals 23. Eine im unteren Teil der Mittelbohrung 12 des Ventilkörpers 1 sichtbare radiale Wirkbohrung 24 endet in der gezeichneten Inhale-Position des Ventilkörpers 1 blind an der hinteren Wand seiner Mittelbohrung 12. Erst wenn die Wirkbohrung 24 des Ventilkörpers 1 an die Wirköffnung 9 des Ventilgehäuses 2 anschließt, was nach einer 90° Drehung im Uhrzeigersinn der Fall ist, ist mit der Exhale-Position (vgl. Fig. 8) die zweite Schaltstellung des Ventilkörpers 1 erreicht, d.h. der Patient steht dann unter einer entsprechenden Sogwirkung der Fördereinheit 10.

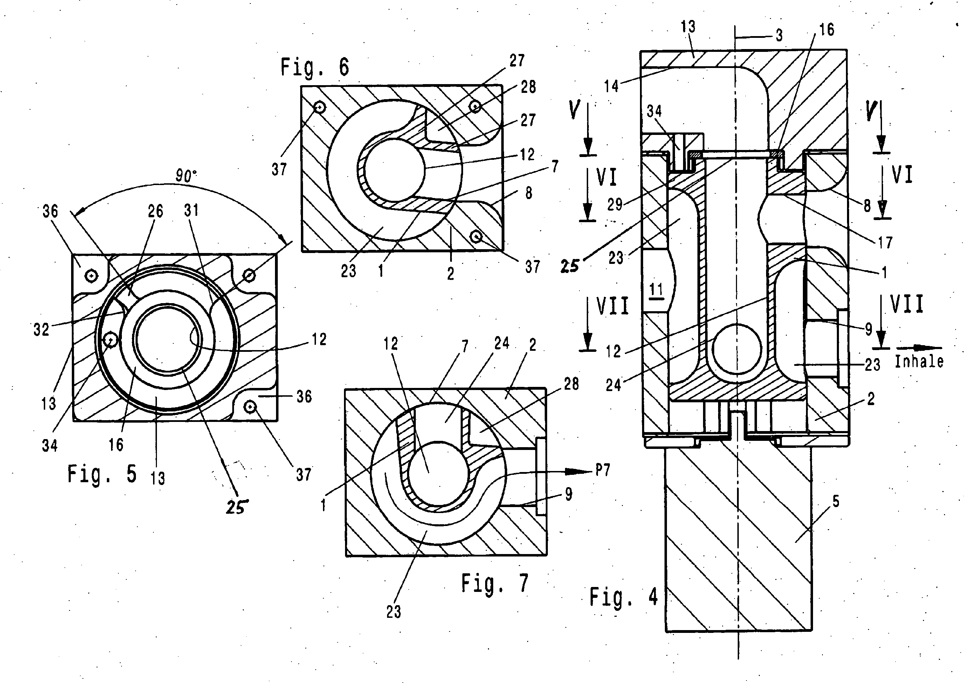

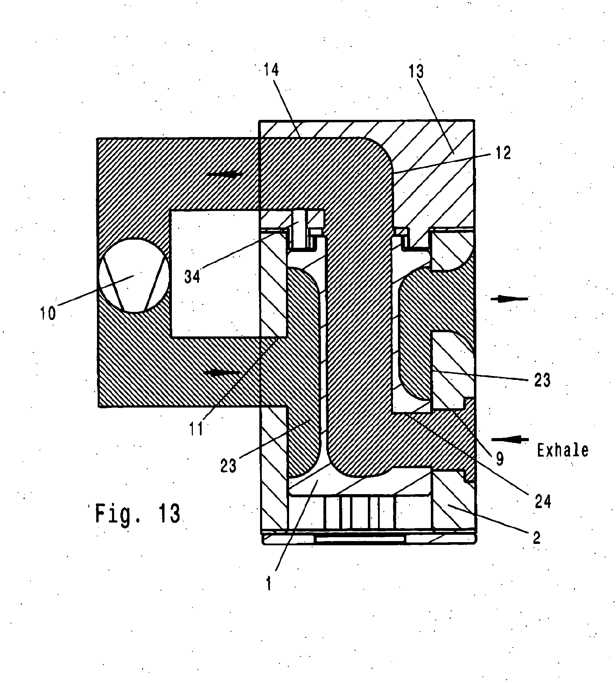

Während die Inhale-Position, wie in Fig. 1 gezeigt in vergrößerter Form den Fig. 4 und 11 entspricht, sei zur Exhale-Position auf die vergrößerten Darstellungen der Fig. 8 und 13 verwiesen.

Um das Therapieziel der Sekretmobilisierung sicherzustellen ist es erforderlich, einen Volumenstrom durch die Fördereinheit 10 zwischen 160 und 300 l/min zu transportieren. Der an die Schlauchleitung 20 über eine Anästhesiemaske angeschlossene Patient erfährt dabei eine maximale Sog-/Druckwirkung, die in ihrer Schwankungsbreite einem starken Hustenreiz entspricht und damit die erwünschte sekretlösende Wirkung zur Folge hat. Das bedeutet im oberen Druckbereich einen Wert von ca. 70 hPa, im unteren Druckbereich von ca. - 70 hPa, wobei das maximale und minimale Druckniveau in den beiden Schaltstellungen des Ventilkörpers 1 sich entsprechend dessen Oszillationsfrequenz ablösen. Die Umschaltzeit zwischen den beiden Schaltstellungen ergibt sich dabei aus einer Oszillationsfrequenz von bis zu 5 Hz. Mit zunehmender Belastbarkeit des Patienten kommen höhere Frequenzwerte im Interesse einer verstärkten Schleimabsonderung infrage. Das vom Fluidstrom erfasste Lungensekret wird in einem (nicht dargestellten) Sekretfilter aufgefangen, der in der zum Patienten führenden Schlauchleitung 20 vorgesehen ist.

Es kann für die Erzielung einer möglichst direkten Druckbeaufschlagungin der Inhale-Position zweckmäßig sein, die druckseitige Anschlussbohrung 11 des Ventilgehäuses 2 aus der Mitte in Uhrzeigerrichtung zu versetzen, d.h. die druckseitige Anschlussbohrung 11 von der Wirköffnung 9 des Ventilgehäuses im Uhrzeigersinn zu entfernen, so dass ein verbessertes tangentiales Einströmen in den Hüllkanal 23 erzielt wird.

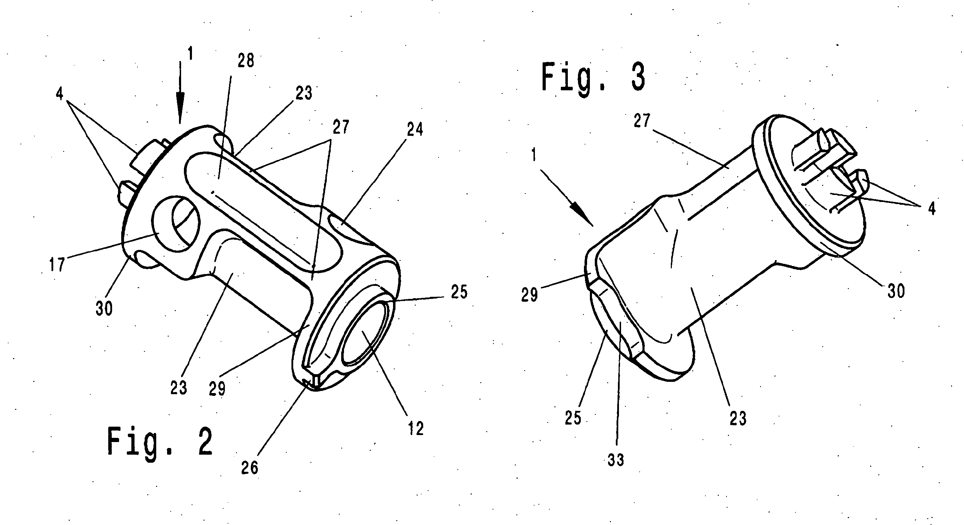

Zur Verbesserung der räumlichen Vorstellung hinsichtlich der Ausbildung des Ventilkörpers ist dieser in den Fig. 2 und 3 aus zwei verschiedenen Ansichten räumlich dargestellt. Dabei sind identische Bauteile mit den gleichen Bezugszeichen wie in Fig. 1 versehen. Man erkennt in Fig. 3 die Einzelheit der Ausbildung der Kupplungsvorsprünge 4 an seiner Unterseite mit Zwischenräumen, in welchen in Fig. 1 nicht näher gezeigte Kupplungsgegenstücke 6 eingreifen, so dass die zwischen Anschlägen alternierende Schaltbewegung des Stellmotors 5 weitgehend spielfrei übertragen wird.

Gemäß Fig. 2 erkennt man auf der den Kupplungsvorsprühgen 4 gegenüberliegenden Seite des Ventilkörpers 1 einen umlaufenden Dichtungssteg 25, an welchem ein Anschlag 26 angeformt ist. Der Anschlag 26 wirkt mit zwei Anschlagsflächen 31, 32 (vgl. Fig. 5) auf Seiten des Gehäusedeckels 13 zusammen, welche der Begrenzung des Schwenkwegs der Schwenkbewegung des Ventilkörpers 1 dienen und auf diese Weise die beiden Schaltstellungen des Ventilkörpers für die Umkehr der Flussrichtung des Fluidstroms bilden. Je nach Schaltstellung des Ventilkörpers 1 wird die Fördereinheit 10 in einer ersten Flussrichtung mit einem Frischfluidkanal 8, 17 und in einer zweiten Flussrichtung mit einem Wirkkanal 9, 24 jeweils saugseitig verbunden, während die druckseitige Verbindung der Fördereinheit gerade umgekehrt erfolgt, nämlich in der ersten Flussrichtung mit dem Wirkkanal 9, 24 und in der zweiten Flussrichtung mit dem Frischfluidkanal 8, 17.

In Fig. 2 erkennt man ferner die maximale umfangsmäßige Erstreckung des Hüllkanals 23 des Ventilkörpers 1 nämlich bis nahe an einen Trennsteg 27, innerhalb dessen ein äußerer Bypass 28 angeordnet ist, welcher sich im wesentlichen über die gesamte Länge des Ventilkörpers 1 erstreckt.

Gemäß Fig. 3 besitzt der obere Scheibenabschnitt 29 des Ventilkörpers 1 eine Aussparung 33, welche in der Bypassposition (vgl. Fig. 12) für den inneren Bypass den Durchfluss des Fluids aus dem Hüllkanal 23 über eine Bypassbohrung 34 im Gehäusedeckel 13 zu dessen Saugleitung 14 freigibt.

Fig. 4 zeigt einen Schnitt durch die Längsachse 3 des Rotationsventils mit Stellmotor 5, wobei der Ventilkörper 1 in die Inhale-Position verschwenkt ist. In dieser Position sind die Frischfluidöffnung 8 des Ventilgehäuses 2 und die Frischfluidbohrung 17 des Ventilkörpers 1 koaxial zueinander eingestellt. Zusammen bilden sie den Frischfluidkanal 8, 17, durch welchen Frischluft über die Mittelbohrung 12 des Ventilkörpers 1 und die Saugleitung 14 von der Fördereinheit 10 (Fig. 1) angesaugt wird. Insoweit kann auf die zu Fig. 1 beschriebene Inhale-Stellung verwiesen werden, wobei gleiche Bauteile mit gleichen Bezugsziffern versehen sind. Die Bypassbohrung 34 für die innere Bypassverbindung ist zum Hüllkanal 23 hin durch den oberen Scheibenabschnitt 29 des Ventilkörpers 1 verschlossen. Die zum Patienten führende Schlauchleitung 20 (Fig. 1) wird druckseitig über den Hüllkanal 23 und die Wirköffnung 9 des Ventilgehäuses 2 mit Druckfluid versorgt.

Die Fig. 5 bis 7 zeigen jeweils Querschnitte durch das Rotationsventil.

Gemäß Fig. 5 erkennt man in der Schnittebene Anschlagsflächen 31, 32 im Gehäusedeckel 13, gegen welche ein an den Ventilkörper 1 angeformter Anschlag 26 abwechselnd anschlägt. Die Ringdichtung 16 liegt auf dem das obere Ende des Ventilkörpers 1 bildenden Dichtungssteg 25 auf, welcher die Mittelbohrung 12 umfasst. Innerhalb dreier Eckausschnitte 36 ist der Gehäusedeckel 13 mittels Schrauben 37 auf dem Ventilgehäuse 1 befestigt.

Die Schnittebene gemäß Fig. 6 zeigt den Ventilkörper 1 mit dem in seinem Außenbereich eingearbeiteten Hüllkanal 23, seiner Mittelbohrüng 12 und seinem Trennsteg 27, in welchem der äußere Bypass 28 eingearbeitet ist.

Die Schnittebene gemäß Fig. 7 zeigt das Ventilgehäuse 2 mit seiner Wirköffnung 9 und im Gehäuseinneren innerhalb der zylindrischen Gehäusebohrung 7 den Ventilkörper 1 mit seinem Hüllkanal 23, seiner Mittelbohrung 12 und seiner gegen die zylindrische Bohrung 7 des Ventilgehäuses 2 blind geschlossenen Wirkbohrung 24. Auch der äußere Bypass 28 ist gegen die zylindrische Bohrung 7 des Ventilgehäuses 2 blind geschlossen.

Die durch den Frischfluidkanal 8, 17 angesaugte Frischluft gelangt über die Mittelbohrung 12 des Ventilkörpers 1 und die Saugleitung 14 des Gehäusedeckels 13 zum saugseitigen Anschluss der Fördereinheit 10 (Fig. 1), deren Druckseite Druckfluid über die druckseitige Anschlussbohrung 11 des Ventilgehäuses 2 in den Hüllkanal 23 liefert. Der Hüllkanal 23 wird gemäß Pfeil P7 durchströmt. Von dort gelangt das Druckfluid durch die Wirköffnung 9 des Ventilgehäuses 2 und über den Patientenschlauch 20 (Fig. 1) zur Lunge des Patienten um diese mit Druckfluid zu beaufschlagen.

Fig. 8 zeigt einen weiteren Axialschnitt durch die Längsachse 3 des Rotationsventils, im Wesentlichen bestehend aus Ventilgehäuse 2 mit Ventildeckel 13, Ventilkörper 1 und Stellmotor 5. Der Ventilkörper 1 befindet sich in seiner Exhale-Position, in welcher die Fördereinheit 10 (Fig. 1) druckseitig an den Hüllkanal 23 über die druckseitige Anschlussbohrung 11 des Ventilgehäuses 2 angeschlossen ist. Der Austritt des Druckfluids erfolgt auf dem Weg gemäß Pfeil P9 (Fig. 9) durch die Frischfluidöffnung 8 des Ventilgehäuses 2. Gleichzeitig wird der Patient über die Schlauchleitung 20 (Fig. 1) abgesaugt, wobei die Ausatemluft durch die Mittelbohrung 12 des Ventilkörpers 1 und weiter durch die Saugleitung 14 des Gehäusedeckels 13 zum saugseitigen Ende der (nicht gezeigten) Fördereinheit 10 geleitet wird. Wie in der Inhale-Position des Ventilkörpers 1 steht dessen Mittelbohrung 12 auch in der Exhale-Position mit dem saugseitigen Eingang der Fördereinheit 10 in Verbindung, d.h. die Mittelbohrung 12 wird stets von einem Saugfluid durchströmt. In der in Fig. 8 gezeigten Exhale-Position gelangt die Ausatemluft des Patienten durch den Wirkkanal 9, 24 in das Innere der Mittelbohrung 12 des Ventilkörpers in den Kreislauf zur Fördereinheit 10 (Fig. 1).

Fig. 10 zeigt eine Schnittebene durch den Wirkkanal 9, 24 mit der Wirköffnung 9 des Ventilgehäuses 2, der Wirkbohrung 24 des Ventilkörpers 1, dessen Mittelbohrung 12 und den Hüllkanal 23.

Auch für die Fig. 8 bis 10 gilt, dass die gleichen Bauteile mit den gleichen Bezugsziffern versehen sind, so dass sich deren wiederholte Nennung erübrigt.

Im vorstehenden Beschreibungsteil wurde das Zusammenwirken des Rotationsventils mit der Fördereinheit 10 erläutert und in diesem Zusammenhang wurden die Bauteile des Rotationsventils in der Inhale-Stellung (Fig. 4) und in der Exhale-Stellung (Fig. 8) für sich und in deren Zusammenwirken beschrieben.

Mit den folgenden Fig. 11 bis 13 wird das Rotationsventil in drei Positionen des Ventilkörpers 1, jeweils mit Einbeziehung der Fördereinheit 10 in den jeweiligen Fluidkreislauf beschrieben. Neben den beiden Wirkpositionen des Ventilkörpers nämlich in der Inhale-Position gemäß Fig. 11 sowie in der Exhale-Position gemäß Fig. 13 wird in der Fig. 12 auch noch die Zwischenstellung des Ventilkörpers 1 in der Übergangsphase zwischen beiden Positionen dargestellt, welche im Folgenden als "Bypass-Position" bezeichnet wird.

In der Fig. 13 ist zur Vereinfachung der Stellmotor weggelassen. Die Strömungswege sind mit engen Schraffuren dargestellt, wobei unterschieden wird zwischen den Strömungswegen auf der Saugseite der Fördereinrichtung 10 und der Druckseite der Fördereinrichtung 10 sowie der in Fig. 12 dargestellten drucklosen Bypassverbindung zwischen Frischfluidöffnung 8 und Wirköffnung 9 des Ventilgehäuses 2.

Gemäß Fig. 12 herrscht in der Bypassposition Druckausgleich zwischen Frischfluidöffnung 8 und Wirköffnung 9; beide Öffnungen werden durch den äußeren Bypass 28 des Ventilkörpers 1 überbrückt. Damit ist eine für den Patienten vorteilhafte Ruhe- bzw. Pausenschaltung gegeben, die insbesondere dann für begrenzte Zeit geschaltet werden kann, wenn der Patient eine Erholungsphase benötigt. Während dieser Erholungsphase kann die Fördereinheit 10 mit einem Mindestfördervolumen weiterlaufen, wobei sich eine Art Kurzschlusskreislauf als innerer Bypass über die Bypassbohrung 34 ausbildet. Das Fluidfördervolumen wird dabei auf einen niedrigen Wert reduziert; eine Ein-/Ausschaltung des gesamten Gerätes wird dabei vermieden, so dass nach Beendigung der Unterbrechung die Behandlung sofort wieder fortgesetzt werden kann.

In der Inhale-Position gemäß Fig. 11 wird saugseitig über die Frischfluidöffnung 8 des Ventilgehäuses 2 Frischluft angesaugt und über den Hüllkanal 23 gemäß Pfeil P7 in Fig. 7 im Bereich der äußeren Hülle des Ventilkörpers 1 um dessen Mittelbohrung 12 herum geführt und über die Wirköffnung 9 des Ventilgehäuses 2 an den Patienten über eine mit diesem verbundenen Schlauchleitung 20 (Fig. 1) weitergeleitet.

Fig. 13 zeigt die der Fig. 8 entsprechende Exhale-Position. Das Sekret des Patienten wird dabei abgesaugt, wobei dessen Ausatemluft über die Wirköffnung 9 des Ventilgehäuses 2 und die Wirkbohrung 24 des Ventilkörpers 1 in dessen Mittelbohrung 12 und von dort über die Saugleitung 14 des Gehäusedeckels 13 mit der Saugseite der Fördereinheit 10 verbunden wird. Druckseitig ist die Fördereinheit mit der druckseitigen Anschlussbohrung 11 des Ventilgehäuses 2 und dem daran angeschlossenen Hüllkanal 23 verbunden, so dass das Druckfluid die Mittelbohrung 12 auf dem Weg gemäß Pfeil P9 der Fig. 9 umströmt und von dort über die Frischfluidöffnung 8 des Ventilgehäuses 2 in die Umgebung verströmt wird.

## Patentansprüche

1. Rotationsventil zur Flussrichtungsumkehr eines druckbeaufschlagten Fluidstroms;
1.a) das Rotationsventil besitzt einen in einem Ventilgehäuse (2) zwischen zwei Schaltstellungen um seine Längsachse (3) verschwenkbar gelagerten Ventilkörper (1);
1.b) eine Fördereinheit für den Fluidstrom ist je nach Schaltstellung des Ventilkörpers (1) saugseitig in einer ersten Flussrichtung mit einem Frischfluidkanal (8, 17) und in einer zweiten Flussrichtung mit einem Wirkkanal (9, 24), druckseitig in der ersten Flussrichtung mit dem Wirkkanal (9, 24) und in der zweiten Flussrichtung mit dem Frischfluidkanal (8, 17) verbunden;
**dadurch gekennzeichnet, dass**:
1.c) der Ventilkörper (1) besitzt um seine Längsachse (3) eine zur Saugseite der Fördereinheit (10) hin offene Mittelbohrung (12), die in beiden Flussrichtungen sogbeaufschlagt ist und die in der ersten Flussrichtung über eine radiale Frischfluidbohrung (17) des Ventilkörpers (1) und in der zweiten Flussrichtung über eine radiale Wirkbohrung (24) des Ventilkörpers (1) nach außen verbunden ist;
1.d) der Ventilkörper (1) besitzt auf seiner Außenseite einen bis auf einen zu seiner Längsachse parallelen Trennsteg (27) umlaufenden Hüllkanal (23), der in beiden Flussrichtungen druckbeaufschlagt ist und der in der ersten Flussrichtung über eine Wirköffnung (9) des Ventilgehäuses (2) und in der zweiten Flussrichtung über eine Frischfluidöffnung (8) des Ventilgehäuses (2) nach außen verbunden ist.

2. Rotationsventil nach Anspruch 1, **dadurch gekennzeichnet, dass** zwischen beiden Schaltstellungen des Ventilkörpers (1) eine Bypassposition vorgesehen ist, wobei ein äußerer Bypass (28) in der Hülle des Ventilkörpers (1) eine Verbindung zwischen der Wirköffnung (9) und der Frischfluidöffnung (8) des Ventilgehäuses (2) und ein innerer Bypass eine Verbindung zwischen dem Hüllkanal (23) und der Saugseite der Fördereinheit (10) bildet.

3. Rotationsventil nach Anspruch 2, **dadurch gekennzeichnet,**
**dass** der äußere Bypass (28) innerhalb des Trennstegs (27) des Ventilkörpers (1) verläuft.

4. Rotationsventil nach Anspruch 1, **dadurch gekennzeichnet, dass** im Bereich beider Stirnseiten des Ventilkörpers (1) Scheibenabschnitte (29, 30) als Führungen für die alternierenden Schwenkbewegungen des Ventilkörpers (1) im Ventilgehäuse (2) vorgesehen sind.

5. Rotationsventil nach Anspruch 1, **dadurch gekennzeichnet, dass** ein Gehäusedeckel (13) des Ventilgehäuses (2) vorgesehen ist, der eine Saugleitung (14) einschließt, welche einerseits dichtend an das zugeordnete offene Ende der Mittelbohrung (12) und andererseits über einen Saugkanal (15) sauseitig an der Fördereinheit (10) angeschlossen ist.

6. Rotationsventil nach Anspruch 5, **dadurch gekennzeichnet, dass** auf der Innenseite des Gehäusedeckels (13) zum Ventilkörper (1) hin vorspringende Anschlagsflächen (31, 32) zur Begrenzung seines Schwenkwegs vorgesehen sind, welche mit einem Anschlag (26) des Ventilkörpers (10) zusammenwirken.

7. Rotationsventil nach Anspruch 1, **dadurch gekennzeichnet, dass** der Ventilkörper (1) mit einem dem offenen Ende gegenüberliegenden Anschlussende mit einem Stellmotor (5) zur Betätigung der Schwenkbewegung für die Flussrichtungsumkehr verbunden ist.

8. Rotationsventil nach Anspruch 4, **dadurch gekennzeichnet, dass** der dem offenen Ende des Ventilkörpers (1) benachbarte Scheibenabschnitt (29) eine Aussparung (33) aufweist, welche in der Bypassposition für den inneren Bypass den Durchfluss des Fluids aus dem Hüllkanal (23) über eine Bypassbohrung (34) im Gehäusedeckel (13) zu dessen Saugleitung (14) freigibt.

9. Rotationsventil nach Anspruch 1, **dadurch gekennzeichnet, dass** Wirkbohrung (24) und Frischfluidbohrung (17) des Ventilkörpers (1) um dessen Schwenkwinkel zwischen beiden Schaltstellungen gegeneinander versetzt sind.

10. Rotationsventil nach Anspruch 1, **dadurch gekennzeichnet, dass** der Schwenkwinkel zwischen beiden Schaltstellungen des Ventilkörpers (1) etwa 90° beträgt.

11. Mechanischer In-/ Exsufflator zur Behandlung von Patienten mit Ateminsuffizienz mit einem Rotationsventil nach einem oder mehreren der Ansprüche 1 bis 10, dessen Ventilgehäuse (2) an den Förderkreislauf einer mit gleichbleibender Drehrichtung rotierenden Fluidfördereinheit (10) angeschlossen ist und dessen Ventilkörper (1) zum Zwecke der Flussrichtungsumkehr mit einstellbarer Frequenz zwischen zwei Schaltstellungen verschwenkbar ist, derart, dass in einer Inhale-Flussrichtung die Saugseite der Fluidfördereinheit (10) mit einer Frischfluidöffnung (8) des Ventilgehäuses (2), deren Druckseite mit einer die Lunge eines Patienten beaufschlagenden Wirköffnung (9) des Ventilgehäuses (2) verbunden ist und
dass in einer Exhale-Flussrichtung die Saugseite der Fluidfördereinheit (10) mit der Wirköffnung (9), deren Druckseite mit der Frischfluidöffnung (8) verbunden ist.

12. Mechanischer In-/ Exsufflator nach Anspruch 11,
**dadurch gekennzeichnet,**
**dass** die Frequenz der Flussrichtungsumkehr bis zu 5 Hz einstellbar ist.

13. Mechanischer In-/ Exsufflator nach Anspruch 11,
**dadurch gekennzeichnet,**
**dass** während der Flussrichtungsumschalung ein innerer Bypass auf "Durchgang" geschaltet ist, derart, dass der Fluidkreislauf durch die Fördereinheit (10) einen definierten Druck aufrecht erhält.

14. Mechanischer In-/ Exsufflator nach Anspruch 13,
**dadurch gekennzeichnet,**
**dass** gleichzeitig mit der Schaltung des inneren Bypass auf "Durchgang" ein äußerer Bypass auf "Öffnen" geschaltet ist, welcher eine Verbindung zwischen der Frischluftöffnung (8) und der Wirköffnung (9) herstellt, derart, dass die mit dem Patienten verbundene Wirköffnung (9) in Verbindung mit der Frischfluidöffnung (8) auf Umgebungsdruck eingestellt ist.

## Claims

1. A rotary valve for the flow direction reversal of a pressurised fluid flow;
1.a) the rotary valve comprises a valve body (1) mounted so as to be swivellable about its longitudinal axis (3) between two switching positions in a valve housing (2);
1.b) depending on the switching position of the valve body (1), a delivery unit for the fluid flow is connected at the suction side to a fresh fluid channel (8, 17) in a first flow direction and to an active channel (9, 24) in a second flow direction, and at the pressure side to the active channel (9, 24) in a first flow direction and to the fresh fluid channel (8, 17) in the second flow direction;
**characterised in that**
1.c) the valve body (1) comprises around its longitudinal axis (3) a central bore (12) open towards the suction side of the delivery unit (10), to which central bore suction is applied in both flow directions and which is connected to the exterior via a radial fresh fluid bore (17) of the valve body (1) in the first flow direction and via a radial active bore (24) of the valve body (1) in the second flow direction;
1.d) at its outer side, the valve body (1) comprises an enveloping channel (23) which is circumferential apart from a separating web (27) parallel to its longitudinal axis, to which enveloping channel pressure is applied in both flow directions and which is connected to the exterior via an active opening (9) of the valve housing (2) in the first flow direction and via a fresh fluid opening (8) of the valve housing (2) in the second flow direction.

2. The rotary valve according to claim 1, **characterised in that** a bypass position is provided between the two switching positions of the valve body (1), wherein an outer bypass (28) in the casing of the valve body (1) creates a connection between the active opening (9) and the fresh fluid opening (8) of the valve housing (2) and an inner bypass creates a connection between the enveloping channel (23) and the suction side of the delivery unit (10).

3. The rotary valve according to claim 2,
**characterised in**
**that** the outer bypass (28) runs inside the separating web (27) of the valve body (1).

4. The rotary valve according to claim 1, **characterised in that**, in the region of the two end faces of the valve body (1), disc sections (29, 30) are provided as guides for the alternating swivelling motions of the valve body (1) in the valve housing (2).

5. The rotary valve according to claim 1, **characterised in that** a housing cover (13) of the valve housing (2) is provided, said housing cover comprising a suction line (14), which on the one hand is connected in a sealing manner to the assigned open end of the central bore (12) and on the other hand is connected via a suction channel (15) on the suction side to the delivery unit (10).

6. The rotary valve according to claim 5, **characterised in that** stop faces (31, 32) projecting towards the valve body (1) are provided on the inner side of the housing cover (13) for limiting its swivel path, said stop faces cooperating with a stop (26) of the valve body (10).

7. The rotary valve according to claim 1, **characterised in that** the valve body (1) is connected, with a connection end lying opposite the open end, to a servomotor (5) for actuating the swivelling motion for the flow direction reversal.

8. The rotary valve according to claim 4, **characterised in that** the disc section (29) adjacent to the open end of the valve body (1) comprises a cutout (33), which in the bypass position for the inner bypass releases the through-flow of the fluid from the enveloping channel (23) via a bypass bore (34) in the housing cover (13) to its suction line (14).

9. The rotary valve according to claim 1, **characterised in that** the active bore (24) and the fresh fluid bore (17) of the valve body (1) are mutually offset about the swivel angle of said valve body between two switching positions.

10. The rotary valve according to claim 1, **characterised in that** the swivel angle between the two switching positions of the valve body (1) amounts to approx. 90°.

11. A mechanical in/exsufflator for the treatment of patients with respiratory insufficiency with a rotary valve according to one or more of claims 1 to 10, the valve housing (2) whereof is connected to the delivery circuit of a fluid delivery unit (10) rotating in a constant rotational direction and the valve body (1) whereof can be swivelled between two switching positions with an adjustable frequency for the purpose of the flow direction reversal, in such a way that, in an inhale flow direction, the suction side of the fluid delivery unit (10) is connected to a fresh fluid opening (8) of the valve housing (2), the pressure side of said fluid delivery unit being connected to an active opening (9) of the valve housing (2) acting on the patient's lungs and
that, in an exhale flow direction, the suction side of the fluid delivery unit (10) is connected to the active opening (9), the pressure side of said fluid delivery unit being connected to the fresh fluid opening (8).

12. The mechanical in/exsufflator according to claim 11, **characterised in**
**that** the frequency of the flow direction reversal can be switched up to 5 Hz.

13. The mechanical in/exsufflator according to claim 11, **characterised in**
**that** during the flow direction switchover, an inner bypass is switched to "passage", in such a way that the fluid circuit through the delivery unit (10) maintains a defined pressure.

14. The mechanical in/exsufflator according to claim 13, **characterised in**
**that** simultaneously with the switching of the inner bypass to "passage", an outer bypass is switched to "open", which creates a connection between the fresh fluid opening (8) and the active opening (9), in such a way that the active opening (9) connected to the patient, in connection with the fresh fluid opening (8), is set to ambient pressure.

## Revendications

1. Soupape rotative pour l'inversion de sens d'écoulement d'un courant fluidique sous pression ;
1.a) la soupape rotative possède un corps de soupape (1) disposé pivotant entre deux positions de commutation sur son axe longitudinal (3) dans un boîtier de soupape (2) ;
1.b) une unité de transport pour le courant fluidique est, en fonction de la position de commutation du corps de soupape (1), reliée côté aspiration à un canal de fluide neuf (8, 17) dans un premier sens d'écoulement et à un canal actif (9, 24) dans un second sens d'écoulement, reliée côté pression au canal actif (9, 24) dans le premier sens d'écoulement et au canal de fluide neuf (8, 17) dans le second sens d'écoulement ;
**caractérisée en ce que**
1.c) le corps de soupape (1) possède sur son axe longitudinal (3) un perçage central (12) ouvrant en direction du côté aspiration de l'unité de transport (10), qui est aspiré dans les deux sens d'écoulement et qui est relié vers l'extérieur par un perçage de fluide neuf radial (17) du corps de soupape (1) dans le premier sens d'écoulement et par un perçage actif (24) du corps de soupape (1) dans le second sens d'écoulement ;
1.d) le corps de soupape (1) possède sur son côté extérieur un canal d'enveloppement circulaire (23) jusqu'à une branche de séparation (27) parallèle à son axe longitudinal, qui est sous pression dans les deux sens d'écoulement et qui est relié vers l'extérieur par une ouverture active (9) du boîtier de soupape (2) dans le premier sens d'écoulement et par une ouverture de fluide neuf (8) du boîtier de soupape (2) dans le second sens d'écoulement.

2. Soupape rotative selon la revendication 1, **caractérisée en ce qu'**une position de dérivation est prévue entre deux positions de commutation du corps de soupape (1), sachant qu'une dérivation extérieure (28) forme, dans l'enveloppe du corps de soupape (1), une liaison entre l'ouverture active (9) et l'ouverture de fluide neuf (8) du boîtier de soupape (2), et une dérivation intérieure forme une liaison entre le canal d'enveloppement (23) et le côté aspiration de l'unité de transport (10).

3. Soupape rotative selon la revendication 2,#
**caractérisée en ce**
**que** la dérivation extérieure (28) passe à l'intérieur de la branche de séparation (27) du corps de soupape (1).

4. Soupape rotative selon la revendication 1, **caractérisée en ce que** des tronçons de disque (29, 30) sont prévus au niveau des deux faces avant du corps de soupape (1) en tant que guides pour les pivotements alternatifs du corps de soupape (1) dans le boîtier de soupape (2).

5. Soupape rotative selon la revendication 1, **caractérisée en ce qu'**un couvercle de boîtier (13) du boîtier de soupape (2) est prévu, qui inclut une ligne d'aspiration (14), laquelle est raccordée d'une part en étanchéité à l'extrémité ouverte correspondante du perçage central (12) et est raccordée côté aspiration d'autre part à l'unité de transport (10) par un canal d'aspiration (15).

6. Soupape rotative selon la revendication 5, **caractérisée en ce que** des surfaces de butée (31, 32) en saillie en direction du corps de soupape (1) pour délimiter le trajet de pivotement sont prévues sur le côté intérieur du couvercle de boîtier (13), lesquelles coopèrent avec une butée (26) du corps de soupape (10).

7. Soupape rotative selon la revendication 1, **caractérisée en ce que** le corps de soupape (1) est relié à une extrémité de raccordement face à l'extrémité ouverte avec un servomoteur (5) pour actionner le pivotement pour l'inversion de sens d'écoulement.

8. Soupape rotative selon la revendication 4, **caractérisée en ce que** le tronçon de disque (29) voisin de l'extrémité ouverte du corps de soupape (1) présente un évidement (33) qui, dans la position de dérivation pour la dérivation intérieure, libère le passage du fluide du canal d'enveloppement (23) par un perçage de dérivation (34) dans le couvercle de boîtier (13) vers sa ligne d'aspiration (14).

9. Soupape rotative selon la revendication 1, **caractérisée en ce que** le perçage actif (24) et le perçage de fluide neuf (17) du corps de soupape (1) sont décalés l'un contre l'autre sur son angle de pivotement entre les deux positions de commutation.

10. Soupape rotative selon la revendication 1, **caractérisée en ce que** l'angle de pivotement entre les deux positions de commutation du corps de soupape (1) fait environ 90°.

11. Insufflateur/exsufflateur mécanique pour le traitement de patient souffrant d'insuffisance respiratoire comprenant une soupape rotative selon l'une des revendications 1 à 10, dont le boîtier de soupape (2) est raccordé au circuit de transport d'une unité de transport de fluide (10) tournant dans un sens de rotation égal, et son corps de soupape (1) peut pivoter entre deux positions de commutation dans le but d'inverser le sens d'écoulement avec une fréquence réglable, de telle sorte que dans un sens d'écoulement d'inhalation, le côté aspiration de l'unité de transport de fluide (10) est relié à une ouverture de fluide neuf (8) du boîtier de soupape (2), dont le côté pression est relié à une ouverture active (9) du boîtier de soupape (2) alimentant le poumon d'un patient et
que dans le sens d'écoulement d'exhalation, le côté aspiration de l'unité de transport de fluide (10) est relié à l'ouverture active (9) dont le côté pression est relié à l'ouverture de fluide neuf (8).

12. Insufflateur/exsufflateur mécanique selon la revendication 11,
**caractérisé en ce**
**que** la fréquence de l'inversion de sens d'écoulement est réglable jusqu'à 5 Hz.

13. Insufflateur/exsufflateur mécanique selon la revendication 11,
**caractérisé en ce**
**que** pendant la commutation de sens d'écoulement, une dérivation intérieure est commutée sur « Passage », de telle façon que le circuit fluidique maintient une pression définie à travers l'unité de transport (10).

14. Insufflateur/exsufflateur mécanique selon la revendication 13,
**caractérisé en ce**
**que** parallèlement à la commutation de la dérivation intérieure sur « Passage », une dérivation extérieure est commutée sur « Ouvrir », laquelle crée une liaison entre l'ouverture de fluide neuf (8) et l'ouverture active (9), de telle sorte que l'ouverture active (9) reliée au patient est réglée à la température ambiante en liaison avec l'ouverture de fluide neuf (8).
